(19) Europäisches Patentamt
European Patent Office
Office européen des brevets

(11) Veröffentlichungsnummer: **0 302 491 A1**

(12) # EUROPÄISCHE PATENTANMELDUNG

(21) Anmeldenummer: **88112703.9**

(51) Int. Cl.4: **C07D 261/08**

(22) Anmeldetag: **04.08.88**

(30) Priorität: **06.08.87 DE 3726127**

(43) Veröffentlichungstag der Anmeldung: **08.02.89 Patentblatt 89/06**

(84) Benannte Vertragsstaaten: **BE CH DE ES FR GB IT LI NL**

(71) Anmelder: **BASF Aktiengesellschaft
Carl-Bosch-Strasse 38
D-6700 Ludwigshafen(DE)**

(72) Erfinder: **Kuekenhoehner, Thomas, Dr.
Seidelstrasse 2
D-6710 Frankenthal(DE)**
Erfinder: **Theobald, Hans, Dr.
Queichstrasse 6
D-6703 Limburgerhof(DE)**
Erfinder: **Goetz, Norbert, Dr.
Schoefferstrasse 25
D-6520 Worms 1(DE)**
Erfinder: **Becker, Rainer, Dr.
Im Haseneck 22
D-6702 Bad Duerkheim(DE)**

(54) **5-Formylisoxazole.**

(57) 5-Formylisoxazole I

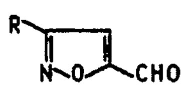

(I)

(R = nicht-aromatischer gesättigter $C_3$-$C_{10}$-Kohlenwasserstoffrest, welcher durch Sauerstoffatome in Ether- oder Acetalfunktion unterbrochen sein kann) sowie die Herstellung von I′

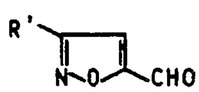

(I′)

(R′ = H, Methyl, Ethyl, R ) durch Oxidation von 5-Hydroxymethylisoxazolen II

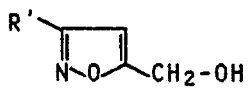

(II)

mit Dimethylsulfoxid in Verbindung mit Oxalylchlorid und anschließender Zugabe einer Base.

EP 0 302 491 A1

Xerox Copy Centre

## 5-Formylisoxazole

Die vorliegende Erfindung betrifft neue 5-Formylisoxazole der allgemeinen Formel I

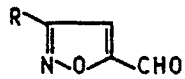 (I)

in der R für einen nicht-aromatischen gesättigten Kohlenwasserstoffrest mit 3 bis 10 C-Atomen steht, welcher durch Sauerstoffatome in Ether- oder Acetalfunktion unterbrochen sein kann, sowie ein Verfahren zur Herstellung von 5-Formylisoxazolen der allgemeinen Formel I′

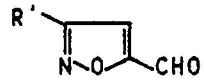 (I′)

in der R′ Wasserstoff, die Methylgruppe, die Ethylgruppe oder einen der Reste R bedeutet, durch Oxidation der entsprechenden 5-Hydroxymethylisoxazole II

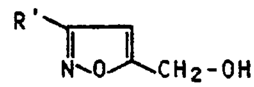 (II)

Zur Herstellung des 3-Methyl-5-formylisoxazols (I′, R′ = Me) sind diverse Wege bekannt, die jedoch nicht befriedigen und sich außerdem zum Teil auch nur schwierig auf die Synthese anderer 3-substituierter 5-Formyl-isoxazole übertragen lassen.

Unter diesen Methoden sind auch solche bekannt, bei denen man von dem leicht zugänglichen 3-Methyl-5-hydroxymethylisoxazol ausgeht und dieses zur entsprechenden Formylverbindung oxidiert.

So verwenden Quilico et al (Gazz. Chim. Ital., Band 69 (1939), Seiten 536-546) Kaliumdichromat als Oxidationsmittel, jedoch erhält man hierbei das 3-Methyl-5-hydroxymethylisoxazol nur in 23%iger Ausbeute, wie, da die Autoren keine Ausbeute angegeben haben, durch eigene Versuche festgestellt wurde.

Auch die Oxidation von 5-Hydroxymethylisoxazol mit Mangandioxid nach Adembri et al (Tetrahedron, Band 23 (1967), Seiten 4697-4707) empfiehlt sich nicht für technische Zwecke, da man hierbei nur 52% der Formylverbindung erhält, wie durch eigene Versuche bestätigt werden konnte.

Weiterhin ist es bekannt, z.B. aus der Arbeit von Omura und Swern (Tetrahedron, Band 34 (1978), Seiten 1651-1660), Alkohole mit einem Gemisch aus Dimethylsulfoxid und Oxalylchlorid zu einem komplexen Addukt und danach mit einer Base umzusetzen, wobei man die den Alkoholen entsprechenden Carbonylverbindungen in meistens hohen Ausbeuten erhält. Diese Untersuchungen mit einem bereits in der Kälte hochwirksamen komplexen Agens erstreckten sich aber nur auf Alkohole mit praktisch inerten Kohlenwasserstoffresten, nicht jedoch auf solche Alkohole, die in ihrem Molekül noch empfindliche Substituenten tragen.

Der Erfindung lag nun die Aufgabe zugrunde, die 5-Formylisoxazole I′ allgemein besser als bisher zugänglich zu machen und neue Verbindungen I aus der Klasse der Verbindungen I′ bereitzustellen.

Demgemäß wurden die eingangs definierten 5-Formylisoxazole gefunden.

Weiterhin wurde ein Verfahren zur Herstellung der ebenfalls eingangs definierten 5-Formylisoxazole I′ durch Oxidation der 5-Hydroxymethylisoxazole II gefunden, welches dadurch gekennzeichnet ist, daß man II bei (-100) bis (+20)°C zunächst mit Dimethylsulfoxid und Oxalylchlorid und danach mit einer Base umsetzt.

Als nicht-aromatische gesättigte Kohlenwasserstoffreste R mit 3 bis 10 C-Atomen in den Verbindungen I bzw. den Ausgangsstoffen II seien genannt:

- lineare Alkylgruppen mit 3 bis 10, vorzugsweise 3 bis 8 C-Atomen, wie die Propyl-, Butyl-, Pentyl-, Hexyl-, Heptyl- und Octylgruppe;
- verzweigte Alkylgruppen mit 3 bis 10, vorzugsweise 3 bis 6 C-Atomen, wie vor allem die Isopropylgruppe und daneben vorzugsweise die Isobutyl-, 2-Butyl-, tert.-Butyl-, 2-Methylbutyl-, 3-Methylbutyl-, 2-Pentyl-, 3-Pentyl- und die 2-Ethylbutylgruppe;

- Cycloalkylgruppen mit 3 bis 10, vorzugsweise 3 bis 7 Ringgliedern, wie die Cyclopropyl-, Cyclobutyl-, Cyclopentyl-, Cyclohexyl- und Cycloheptylgruppe;
- alkylsubstituierte Cycloalkylgruppen mit 4 bis 10, vorzugsweise 6 bis 8 C-Atomen, darunter vor allem die Methylhomologen der Cycloalkylgruppen, wie die 1-, 2- und 3-Methylcyclopentyl-, 1-, 2-, 3- und 4-Methylcyclohexyl- und die 1-, 2-, 3- und 4-Methylcycloheptylgruppen;
- cycloalkylsubstituierte Alkylgruppen mit 4 bis 10, vorzugsweise 4 bis 8 C-Atomen, darunter vorzugsweise solche, in denen die Cycloalkylgruppe 4 bis 7 Ringglieder hat und die Alkylenbrücke 1 oder 2 C-Atome aufweist, wie beispielsweise die Cyclopropylmethyl-, 1- und 2-Cyclopropylethyl-, Cyclobutylmethyl-, 1- und 2-Cyclobutylethyl-, Cyclopentylmethyl-, 1- und 2-Cyclopentylethyl-, Cyclohexylmethyl-, 1- und 2-Cyclohexylethyl-, Cycloheptylmethyl- und die 1- und 2-Cycloheptylethylgruppe.
- durch Sauerstoffatome in Etherfunktion unterbrochene Alkylreste mit vorzugsweise 3 bis 8 C-Atomen, wie die 2-Methoxyethylgruppe, die 1-Methoxyethylgruppe, die 2-Ethoxyethylgruppe, die 1-Ethoxyethylgruppe, die 3-Methoxypropylgruppe, die 1-Methoxyprop-2-ylgruppe und die 1,3-Dimethoxyprop-2-ylgruppe;
- Alkoxycycloalkylreste mit vorzugsweise 3 bis 8 Ringgliedern und 1 bis 4 C-Atomen in den Seitenketten, wie die 2- oder 3-Methoxycyclopentylgruppe, die 2-, 3- oder 4-Methoxycyclohexylgruppe, die 2,3-, 2,4-, 2,5- oder 3,4-Dimethoxycyclopentylgruppe und die 2-, 3- oder 4-Methoxycyclohexylgruppe;
- durch Sauerstoffatome in Acetalfunktion unterbrochene Alkylgruppen mit vorzugsweise 3 bis 8 C-Atomen, wie die Dimethoxymethylgruppe, die Diethoxymethylgruppe und die 2,2-Dimethoxyethylgruppe;
- durch Ringsauerstoffatome in Etherfunktion unterbrochene Cycloalkylreste mit vorzugsweise 5 oder 6 Ringgliedern, wobei diese Cycloalkylreste ihrerseits $C_1$-$C_4$-Alkylgruppen tragen können, beispielsweise die Tetrahydrofuran-2-ylgruppe, die Tetrahydrofuran-3-ylgruppe, die Tetrahydropyranylgruppe mit 2-, 3- oder 4-Verknüpfung, die 1,4-Dioxanylgruppe sowie die verschiedenen Methyl- und Ethylderivate dieser Gruppen;
- durch Ringsauerstoffatome in Acetalfunktion unterbrochene Cycloalkylreste oder Alkylcycloalkyl mit vorzugsweise 5 oder 6 Ringgliedern, wobei diese Cycloalkylreste ihrerseits $C_1$-$C_4$-Alkylgruppen tragen können, beispielsweise die Dioxolan-2-ylgruppe, die Dioxolan-4-ylgruppe, die Dioxolan-2-ylmethylgruppe, die 2-(Dioxolan-2-yl)-ethylgruppe, die 4-Methyldioxolan-2-ylgruppe und die entsprechenden vom 1,3-Dioxan abgeleiteten Verbindungen.

Soweit es sich um sauerstoffhaltige Reste R handelt, enthalten diese vorzugsweiser ein oder zwei Sauerstoffatome in Etherfunktion oder zwei Sauerstoffatome in Acetalfunktion.

Bezüglich des Herstellungsverfahrens kommen als weitere Reste R′ in den Ausgangsverbindungen II bzw. den Verfahrensprodukten I′ noch Wasserstoff, die Methylgruppe und die Ethylgruppe hinzu.

Die Ausgangsverbindungen II sind entweder bekannt oder nach bekannten Methoden erhältlich, wobei sich die Herstellung aus Nitriloxiden und Propargylalkohol

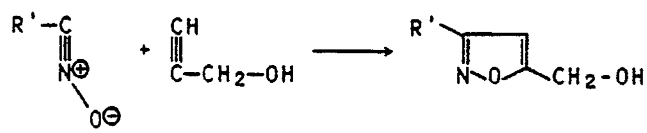

besonders empfiehlt. Die Nitriloxide sind ihrerseits durch Umsetzung der Aldehyde R′-CHO mit Hydroxylamin zu den Aldoximen R′-C=N-OH und Oxidation der Aldoxime mit Hypochloriten erhältlich. Näheres zu diesen Reaktionen ist der DE-A 2 754 832 zu entnehmen.

Das oxidierende Agens beim erfindungsgemäßen Verfahren ist Dimethylsulfoxid ($CH_3$-SO-$CH_3$), welches mit Oxalylchlorid (Cl-CO-CO-Cl) aktiviert ist und in Form des intermediär gebildeten Sulfoniumsalzes

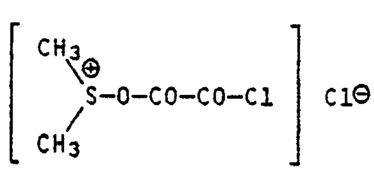

seine Wirksamkeit entfaltet, wobei man zweckmäßigerweise pro Mol Oxalylchlorid 1 bis 10 mol Dimethylsulfoxid einsetzt. Die Menge des Oxalylchlorids beträgt vorteilhaft 1 bis 2 mol pro Mol II.

Im Bereich der Reaktionstemperaturen von (-100) bis (+20)° C empfehlen sich besonders Temperaturen zwischen (-70) und 0° C.

Nach Bildung des Addukts versetzt man das Reaktionsgemisch mit einer Base, wobei die Carbonylverbindungen I′ entstehen. Als Basen eignen sich Mineralbasen wie Natriumhydroxid, Alkoholate wie Natriummethylat, besonders aber tertiäre Stickstoffbasen wie Pyridin und Triethylamin.

Die Menge der Base beträgt zweckmäßigerweise 1 bis 10 mol pro Mol II.

In aller Regel empfiehlt sich die Mitverwendung eines inerten und - im Hinblick auf die Aufarbeitung - wasserunlöslichen Lösungsmittels, wie Toluol und Chloroform sowie vor allem Methylenchlorid in Mengen von etwa 5 bis 50 l pro kg II.

Man führt die Umsetzung zweckmäßigerweise so aus, daß man eine Lösung des Oxalylchlorids vorlegt und hierzu bei der gewählten Reaktionstemperatur allmählich eine Lösung des Dimethylsulfoxids gibt, oder umgekehrt. Das so hergestellte oxidierende Agens wird dann allmählich mit einer Lösung von II versetzt, wobei die umgekehrte Zugabe ebensogut möglich ist.

Nach dieser Reaktion, die trotz der niedrigen Temperaturen sehr rasch verläuft, fügt man die Base hinzu, läßt erwärmen, versetzt die Mischung mit Wasser und arbeitet die organische Phase wie üblich - in der Regel destillativ - auf das 5-Formylisoxazol auf.

Die Formylisoxazole I′ sind wertvolle Zwischenprodukte für organische Synthesen, z.B. für die Herstellung von Pflanzenschutzmitteln, Farbstoffen und Arzneimitteln. Unter den neuen Verbindungen I kommt dem 3-Isopropyl-5-formylisoxazol, welches zur Herstellung von Herbiziden dient, wie sie in der älteren deutschen Anmeldung P 36 09 181.2 beschrieben sind, besondere Bedeutung zu.

Beispiele 1-16

Eine Lösung aus 14 g (0,11 mol) Oxalylchlorid und 250 ml Methylenchlorid wurde bei (-60)°C allmählich mit einer Lösung aus 18,7 g (0,24 Mol) Dimethylsulfoxid und 50 ml Methylenchlorid und danach allmählich mit einer Lösung aus 0,10 mol eines durch den Rest R′ charakterisierten 5-Hydroxymethylisoxazols II

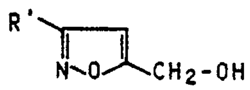

(II),

und 30 ml Methylenchlorid versetzt und noch 15 Minuten bei (-60)°C gehalten.

Danach wurden 50,5 g (0,5 mol) Triethylamin zugetropft, und anschließend wurden bei Raumtemperatur 300 ml Wasser zu dem Reaktionsgemisch gegeben.

Die wäßrige Phase wurde abgetrennt und noch dreimal mit Methylenchlorid extrahiert, wonach die vereinigten Methylenchloridphasen zunächst mit verdünnter Salzsäure und sodann mit $NaHCO_3$-Lösung gewaschen und wie üblich destillativ auf die Verfahrensprodukte I′ aufgearbeitet wurden, die in einer Ausbeute von y% anfielen.

Im Falle nicht-destillierbarer Produkte wurden die Ausbeuten an von Methylenchlorid befreitem Rohprodukt angegeben, wobei die Reinheit in diesen Fällen gemäß gaschromatographischer Analyse mindestens 95% betrug.

Die Produkte wurden durch $^1H$-NMR-Spektralanalyse sowie meistens auch durch ihre Siede- und Schmelzpunkte identifiziert.

Einzelheiten zu diesen Versuchen sind der folgenden Tabelle zu entnehmen.

| Bsp. | R′ | Kp. [°C/mbar] Fp. [°C] | y [%] | $^{1}H$-NMR-Spektrum ($CDCl_3$) δ/ppm = |
|---|---|---|---|---|
| 1 | Methyl | 46°C | 75 | 2,4 (s,3H); 6,9 (s,1H); 9,9 (s,1H); |
| 2 | Ethyl | 85-90°C/4,4 mbar | 82 | 1,3 (t,3H); 2,8 (q,2H) 6,9 (s,1H); 10,0 (s,1H); |
| 3 | Propyl | 90°C/4 mbar | 88 | 1,0 (t,3H); 1,7 (m,2H); 2,8 (t,2H); 6,9 (s,1H); 10,0 (s,1H); |
| 4 | Isopropyl | 70-74°C/4 mbar | 83 | 1,4 (d,6H); 3,2 (hept., 1H) 7,0 (s,1H); 10,0 (s,1H); |
| 5 | Isobutyl | 115°C/4,0 mbar | 85 | 1,0 (d,6H); 2,0 (m,1H); 2,7 (d,3H); 6,8 (s,1H) 10,0 (s,1H); |
| 6 | tert.Butyl | - | 88 | 1,4 (s,9h); 6,9 (s,1H); 10,0 (s,1H); |
| 7 | Pentyl | 70-73°C/0,1 mbar | 85 | 0,8-1,1 (m,3H); 1,2-1,4 (m,4H); 1,5-1,8 (m,2H); 2,8 (t,2H); 6,9 (s,1H); 10,0 (s,1H); |
| 8 | Heptyl | 83-90°C/0,2 mbar | 86 | 0,8-1,9 (m,13H); 2,8 (t,2H); 6,9 (s,1H); 10,0 (s,1H); |
| 9 | Cyclopropyl | 75-80°C/1,5 mbar 45-51°C | 84 | 0,7-1,3 (m,4H); 2,0-2,2 (m,1H); 6,7 (s,1H); 9,9 (s,1H); |
| 10 | Cyclobutyl | 93-98°C/3 mbar | 89 | 1,7-2,6 (m,6H); 3,5-3,8 (m,1H); 6,9 (s,1H); 9,9 (s,1H); |
| 11 | Cylcopentyl | 75°C/0,1 mbar | 88 | 1,5-2,3 (m,8H); 3,1-3,4 (m,1H); 6,9 (s,1H); 10,0 (s,1H); |
| 12 | Cylcohexyl | | 94 | 1,1-2,1 (m,10H); 2,6-2,9(m,1H); 6,9 (s,1H); 10,0 (s,1H); |
| 13 | Tetrahydrofuran-2-yl | 86°C/0,01 mbar | 90 | 1,9-2,2 (m,3H); 2,2-2,6(m,1H); 3,8-4,1 (m,2H); 5,0-5,2(m,1H); 7,0 (s,1H); 10,0 (s,1H); |
| 14 | Tetrahydrofuran-3-yl | 106°C/0,1 mbar | 87 | 2,0-2,2 (m,1H); 2,3-2,6(m,1H); 3,5-3,8 (m,1H); 3,8-4,2(m,4H); 7,0 (s,1H); 10,0 (s,1H); |
| 15 | Tetrahydropyran-2-yl | 95°C/0,01 mbar | 82 | 1,5-1,9 (m,4H); 1,9-2,2(m,2H); 3,5-3,8 (m,1H); 4,0-4,2(m,1H); 4,5-4,7 (m,1H); 7,1 (s,1H); 10,0 (s,1H); |
| 16 | Tetrahydropyran-3-yl | 95°C/0,05 mbar | 88 | 1,5-2,0 (m,3H); 2,0-2,3(m,1H); 3,0-328 (m,1H); 3,4-3,7(m,2H); 3,8-4,2 (m,2H); 7,0 (s,1H); 10,0 (s,1H); |

## Ansprüche

1. 5-Formylisoxazole der allgemeinen Formel I

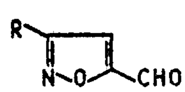

(I)

in der R für einen nicht-aromatischen gesättigten Kohlenwasserstoffrest mit 3 bis 10 C-Atomen steht, welcher durch Sauerstoffatome in Ether- oder Acetalfunktion unterbrochen sein kann.

2. 5-Formylisoxazole gemäß Anspruch 1, in denen R eine lineare $C_3$-$C_8$-Alkylgruppe bedeutet.

3. 5-Formylisoxazole gemäß Anspruch 1, in denen R eine methyl- oder ethylverzweigte Alkylgruppe mit 3 bis 8 C-Atomen bedeutet.

4. 3-Isopropyl-5-formylisoxazol.

5. 5-Formylisoxazole gemäß Anspruch 1, in denen R eine Cycloalkylgruppe mit 3 bis 7 Ringgliedern bedeutet.

6. Verfahren zur Herstellung von 5-Formylisoxazolen der allgemeinen Formel I′

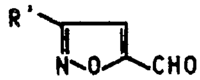

(I′)

in der R′ für Wasserstoff, die Methylgruppe, die Ethylgruppe oder einen der Reste R gemäß Formel I in Anspruch 1 bedeutet, durch Oxidation der entsprechenden 5-Hydroxymethylisoxazole II

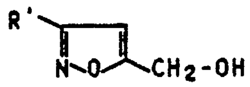

(II)

dadurch gekennzeichnet, daß man II bei (-100) bis (+20)°C zunächst mit Dimethylsulfoxid und Oxalylchlorid und danach mit einer Base umsetzt.

Europäisches Patentamt

# EUROPÄISCHER RECHERCHENBERICHT

Nummer der Anmeldung

EP 88112703.9

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | DE - A1 - 2 909 025 (TAIHO)<br>* Formel II ; Seite 7, Zeile 13 *<br>-- | 1,2,3,4 | C 07 D 261/08 |
| X | CHEMICAL ABSTRACTS, Band 91, Nr. 25, 17. Dezember 1979, Columbus, Ohio, USA<br>MANCUSO, ANTHONY J. et al. "Structure of the dimethyl sulfoxide-oxalyl chloride reaction product. Oxidation of heteroaromatic and diverse alcohols to carbonyl compounds"<br>Seite 595, Spalte 2, Zusammenfassung-Nr. 210 384h<br>& J.Org.Chem. 1979, 44(23), 4148-50<br>-- | 6 | |
| A | CHEMICAL ABSTRACTS, Band 91, Nr. 7, 13. August 1979, Columbus, Ohio, USA<br>HONNA, RYUJI et al. "2-Guanidino-4-(5H)-imidazolone derivatives"<br>Seite 709, Spalte 2, Zusammenfassung-Nr. 57 006q<br>& Jpn. Kokai Tokkyo Koho 79 19,977<br>-- | 1 | RECHERCHIERTE SACHGEBIETE (Int. Cl.4)<br>C 07 D 261/00 |

Der vorliegende Recherchenbericht wurde für alle Patentansprüche erstellt.

| Recherchenort | Abschlußdatum der Recherche | Prüfer |
|---|---|---|
| WIEN | 29-09-1988 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

Europäisches Patentamt

# EUROPÄISCHER RECHERCHENBERICHT

Nummer der Anmeldung

-2-

EP 88112703.9

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | CHEMICAL ABSTRACTS, Band 92, Nr. 7, 18. Februar 1980, Columbus, Ohio, USA<br>HAYASHI, S.I. et al. "The behavior of 1-(5-oxazolyl)-1-alkylidenes and 1-(5-isoxazolyl)-1-alkylidenes" Seite 663, Spalte 2, Zusammenfassung-Nr. 58 661g<br>& Tetrahedron Lett. 1979, (32), 2961-4<br>-- | 1 | |
| A | CHEMICAL ABSTRACTS, Band 100, Nr. 13, 26. März 1984, Columbus, Ohio, USA<br>MARX, MICHAEL et al. "Reactivity-selectivity in the Swern oxidation of alcohols using dimethyl sulfoxide-oxalyl chloride" Seite 687, Spalte 2, Zusammenfassung-Nr. 103 729g<br>& J. Org. Chem. 1984, 49(5), 788-93<br>-- | 6 | RECHERCHIERTE SACHGEBIETE (Int. Cl.4) |
| A | CHEMICAL ABSTRACTS, Band 89, Nr. 3, 17. Juli 1978, Columbus, Ohio, USA<br>MANCUSO, ANTHONY J. et al. "Oxidation of long-chain and related alcohols to carbonyls by dimethyl sulfoxide "activated" by oxalyl chloride" Seite 602, Spalte 2, Zusammenfassung-Nr. 23 690b<br>& J. Org. Chem. 1978, 43(12), 2480-2<br>---- | 6 | |

Der vorliegende Recherchenbericht wurde für alle Patentansprüche erstellt.

| Recherchenort | Abschlußdatum der Recherche | Prüfer |
|---|---|---|
| WIEN | 29-09-1988 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82